# EUROPEAN PATENT SPECIFICATION

(11) **EP 1 736 206 B1**
(45) Date of publication and mention of the grant of the patent: **19.10.2011**
(21) Application number: 05710527.2
(22) Date of filing: 16.02.2005
(51) Int. Cl.: A61K 36/07, A61K 36/074, A61K 36/25, A61P 35/00

(54) **PHYSIOLOGICALLY ACTIVE COMPOSITION AND PROCESS FOR PRODUCING THE SAME**
PHYSIOLOGISCH WIRKSAME ZUSAMMENSETZUNG UND VERFAHREN ZU IHRER HERSTELLUNG
COMPOSITION PHYSIOLOGIQUE ACTIVE ET SON PROCESSUS DE PRODUCTION

(30) Priority: 16.02.2004 JP 2004038443
(43) Date of publication of application: 27.12.2006
(73) Proprietor: Goino, Tadashi, Hotaka Azumino-shi Nagano 399-8301 (JP)
(72) Inventor: Goino, Tadashi, Hotaka Azumino-shi Nagano 399-8301 (JP)
(74) Representative: Benson, John Everett
(86) International application number: PCT/JP2005/002824
(87) International publication number: WO 2005/077395

(56) References cited:
- EP-A- 1 908 474
- WO-A2-01/56589
- JP-A- 11 292 786
- JP-A- 2000 143 526
- JP-A- 2002 000 229
- JP-A- 2002 235 084
- JP-A- 2002 315 570
- JP-A- 2003 189 817
- JP-A- 2003 342 198
- WINTHER K ET AL: "A combination of Japanese ginseng, Ganoderma lucidum and Trametes versicolor, referred to as the Goino procedure, can lower blood glucose and LDL-cholesterol in patients with NIDDM" ATHEROSCLEROSIS SUPPLEMENTS, ELSEVIER, vol. 4, no. 2, 1 September 2003 (2003-09-01), page 339, XP009118160 ISSN: 1567-5688
- YAMAUCHI K. ET AL: 'Tanshi Kinrui no Kosanka Sayo to sono Denki Kagakuteki Kento (Electrochemical Method for Estimating Antioxidative Activities of Fungi).' J.TECHNOLOGY AND EDUCATION. vol. 10, no. 2, 2003, pages 81 - 86, XP002995762

## Description

### Technical Field

The present invention relates to a composition having physiological activity such as an antitumor effect and a hypoglycemic effect. Specifically, it relates to a physiologically active composition including an extract composition from more than two kinds of fungi selected from a fungi belonging to the Basidiomycetes and one from a root of a plant belonging to Araliaceae as described below.

### Background Art

It has been known from the past that a carpophore or a mycelium culture (including a mixture of a mycelium and a culture in addition to the mycelium alone and hereinafter these are simply referred to as a culture) of a varnished conk belonging to Basidiomycetes is a herbal medicine and has various medical benefits and a component contained therein, including the polysaccharides, has a certain type of antitumor effect. Similarly, various physiologically active substances including one with the antitumor activity are also extracted from a carpophore of Turkey Tail belonging to Basidiomycetes. Furthermore, a root a plant belonging to Araliaceae including ginseng and an extract composition thereof have been known from the past as a herbal medicine and many medical benefits thereof are also known. It is also known that a carpophore of Phellinus linteus belonging to Phellinus as Basidiomycetes and that of Agarics belonging to Agaricaceae contain useful saccharides such as B-glucan. (Japanese Published

Unexamined Patent Application No. 2003-183176).

### Disclosure of the Invention

An effective component such as an antitumor active component in the above described herbal medicines has not been elucidated in detail yet. Effectiveness and synergistic effects in a mixture of these herbal medicines have also not been scientifically elucidated yet so that prediction of its effectiveness is difficult.

The present inventor has found that a composition formulated with an extract component derived from several fungi belonging to Basidiomycetes and an extract component derived from a root of a plant belonging to Araliaceae as described below show an oxidation-reduction potential, of which the magnitude expresses physiological activity such as the antitumor effect and lowering of blood glucose levels (hereinafter this effect is referred to as the hypoglycemic effect) in a hyperglycemic patient and completed the present invention.

The present invention is directed to a physiologically active composition containing: an extract component from a carpophore of the following Basidiomycetefungi:Ganoderma lucidium,CoriolusVersicolor and Phellinus linteus; and an extract component from a root of P. japonicus C.A. Meyer belonging to Araliaceae, wherein the ratio of Ganoderma Lucidum: Coriolus Versicolor: Phellinus Linteus: P. japonicus C.A. Meyer in the raw material for extraction is 1:1:1:1 by weight, and wherein the oxidation-reduction potential of the aqueous solution of the composition is not more than 330 mV.

The pH of an aqueous solution of the composition is preferably not less than 4.5 and not more than 6.5.

In the composition of the invention, the extract composition from a carpophore of the Basidiomycete fungi and the extract component from a root of a plant belonging to Araliaceae are respectively extract components with hot water.

The present invention is also directed to a drug formulation which is mixed when used and is provided with a first drug comprising a hot water extract component from a carpophore, a mycelium and a culture of Ganoderma lucidum, Coriolus Versicolor and Phellinus linteus, and a second drug comprising a hot water extract component from a root of P. japonicus C.A. Meyer, wherein the ratio of Ganoderma Lucidum: Coriolus Versicolor: Phellinus Linteus: P. japonicus C.A. Meyer in the raw material for extraction is 1:1:1:1 by weight, and wherein the oxidation-reduction potential of an aqueous solution of the formulation is not more than 330 mV.

The present invention is further directed to a preparing method of a physiologically active composition, comprising the steps of; extracting with hot water a carpophore of Gandoderma lucidium and Coriolus Versicolor; extracting with hot water a carpophore of Phellinus linteus; extracting a root of P. japonicuous C.A. Meyer with hot water; wherein the ratio of Ganoderma Lucidum: Coriolus Versicolor: Phellinus Linteus: P. japonicus C.A. Meyer in the raw material for extraction is 1:1:1:1 by weight, and mixing the extract components with the hot water obtained in the extracting steps.

The present invention is further directed to a preparing method of the physiologically active composition, comprising the steps of: obtaining a hot water extract from a carpophore of Ganoderma lucidium and Coriolus Versicolor, a hot water extract from a carpophore of Phellinus linteus, and a hot water extract from a root of P. japonicus C.A. Meyer separately or from the same extraction system with respect to at least more than two kinds of the hot water extracts, wherein the ratio of Ganoderma Lucidum: Coriolus Versicolor: Phellinus Linteus: P. japonicus C.A. Meyer in the raw material for extraction is 1:1:1:1 by weight, so as to prepare a composition comprising the hot water extract component from a carpophore of Ganoderma lucidium and Coriolus Versicolor, the hot water extract component from a carpophore of Phellinus linteus and the hot water extract component from a root of P. japonicus C.A. Meyer.

### Best Mode for Carrying Out the Invention

The best mode for carrying out the present invention is described below.

### [Fungus belonging to Basidiomycetes Aphyllophorales Ganoderma Ganodermaceae and fungus belonging to Aphyllophoarles Polyporaceae Coriolus]

In the present invention, an extract obtained from the carpophore of more than two kinds of the fungi belonging to these genera is contained as an active component. The fungi belonging to Ganodermaceae and Coriolous are used in combination.

In Ganodermaceae, Reishi Fungus (Ganoderma Lucidum) is used. Reishi Fungus is preferably bred by nature on trees, but is rarely autogenous. Reishi Fungus can be artificially cultivated. Reishi Fungus has a shiny waxy cap section and a stem section, the length of which reaches up to approximately 15 cm. The color of the carpophore is red, blue, yellow, white, purple or black. This fungus grows on a stump or near the base of a tree weakened by disease to form a white protonema.

Coriolus Versicolor is used as Coriolus. Coriolus Versicolor naturally grows in the western part of Japan, particularly in the Shinshu (specifically, Nagano Prefecture), Shikoku and Kyushu regions. Coriolus Versicolor is a xylophilous fungus and particularly xylophilous to a broadleaf tree.

### [Fungus belonging to Basidiomycetes Agaricales Hymenochaetaceae Phellinus]

Phellinus linteus is used as the Basidiomycete fungus belonging to Phellinus.

Natural Phellinus linteus is a wooden fungus in a brilliant yellow or brownish yellow color to parasitize a mulberry and generate a semicircular carpophore. In Chinese medicine, this fungus is referred to as Sang-Hwang.

Basidiomycetes are not particularly limited to, but may include fungi naturally grown in the wild, fungi artificially cultivated, or fungi cell-cultured. The fungi naturally grown in wild are preferable. The carpophore is preferably air-dried at an ambient temperature under light shielding.

Particularly with the Ganodermaceae fungus, the carpophore of the black color naturally matured is preferably used. With the Coriolus fungus, the autogenous carpophore picked in summer is preferable and more preferably air-dried at an ambient temperature under light shielding.

### [Root of Araliaceae Plant]

The composition of the present invention also includes an extract composition from a root of a plant belonging to Araliaceae as the active component. Japanese ginseng (Zhuzishen ginseng, P. Japonicus C. A. Meyer), is used as the Araliaceae plant. The root is used.

### [Preparing method of the Composition]

The composition of the present invention is obtained by first extracting with hot water the Basidiomycete fungi including the Ganodermaceae, Coriolus and Phellinus fungi described above and the root of anAraliaceae plant described above. The raw material component in the composition comprises the combination of the fungi described above. Each raw material or a combination of more than two kinds of the raw materials may be extracted with hot water to obtain the Basidiomycete fungi and Araliaceae plant root extract components separately, which may then be mixed. Or both Basidiomycete fungi and a root from an Araliaceae plant as the raw material are extracted together with hot water. Preferably, all raw materials for the extract component to be contained in the composition are mixed and then extracted with hot water. In extraction with hot water, each raw material is preferably crushed into small pieces or powder and more preferably into small pieces. Crushing into small pieces of a cube roughly 5 mm or shorter on the side is particularly preferable.

In the raw material for extraction, the amount of Basidiomycetes and that of the root of an Araliaceae plant in a genus unit used are equal to each other in weight. For example, when the Ganodermaceae, Coriolus and Phellinus fungi as the Basidiomycete fungus and the Japanese ginseng as the Araliaceae plant are used, an equal amount, for example, 4 to 7 g of each material can be extracted with 1000 ml of hot water.

When the composition is prepared with the raw material for extraction in a formulation ratio (weight ratio) of 1:1:1:1 of Ganodermaceae carpophore: Coriolus carpophore: Phellinus carpophore: Japanese ginseng (root), which is a composition of the present invention, for example, 6 g of the Ganodermaceae carpophore, 6 g of the Coriolus carpophore, 6 g of the Phellinus carpophore and 6 g of the Japanese ginseng are picked, mixed and crushed into small cubed pieces roughly 5 mm on the side, to which 500 to 1000 ml of distilled water is added to boil for three hours after by using a reflux condenser and then filtered to yield the composition (stock solution) used in the present invention.

The temperature of the hot water for extraction is from 80°C to 100°C, preferably from 90°C to 95°C. The extraction time is preferably at least one hour, more preferably longer than 2 hours, further preferably longer than 2.5 hours. An upper limit for the extraction time is 3 to 4 hours. The extraction operation is preferably carried out using a reflux condenser. An amount of water to the raw material for extraction is not particularly limited, but 500 parts by weight to 1000 parts by weight of water is preferably used to extract 10 parts by weight to 30 parts by weight of the raw material. An extracted liquid obtained in extraction at this weight ratio (particularly in the case where the reflux condenser is used) forms a liquid with a concentration suitable for administration without modification.

The obtained extracted liquid is filtered to remove the residual raw material for extraction. A filtrate or supernatant liquid of the extracted liquid is concentrated to yield a concentrated liquid if needed. Water in the extracted liquid can be evaporated to yield a solid (powder-like) extract component, and the extract component is dried to yield a desired dry extract component if needed.

Furthermore, when considering the dosing manner and dosage form of the composition, an additive for the drug preparation or stabilization of the extract component can be added during concentration or drying.

Furthermore, a stock solution of this composition can be properly diluted and used as the composition of the present invention. A dilution ratio can be set as needed and the stock solution is diluted roughly in a range of twofold to three hundredfold. Preferably the dilution ratio is used from twofold to twenty fold, more preferably from fourfold to sixteen fold. A medium for dilution is not particularly limited, but is preferably water.

A solvent in this stock solution can also be removed by a proper method for drying to obtain the above-described solid extract component, which is used as the composition of the present invention.

The composition can be composed as drug products, which are mixed when used. That is, a first drug containing the extract component from Basidiomycete and a second drug containing the extract component from the root of a plant belonging to Araliaceae can be combined for mixing when used. Each of the first and second drugs may be in a form of an aqueous solution or a solid such as powder.

The thus prepared composition is used when the oxidation-reduction potential of its solution is lower than 330 mV.

In particular, the composition with this range of the oxidation-reduction potential is preferably used as the antitumor active composition and hypoglycemic agent composition. The oxidation-reduction potential is measured in an aqueous solvent, preferably in water. The stock solution obtained by the extraction or its aqueous diluted solution can be used without change to measure the oxidation-reduction potential. When the composition is solidified, it is dissolved or suspended with a proper solvent or water for measurement.

The oxidation-reduction potential of a solution of the composition is preferably lower than 300 mV, further more preferably lower than 250 mV. Most preferably it is below 0 mV. The oxidation-reduction potential is preferably higher than -1200 mV, more preferably higher than -300 mV.

A pH value of a solution of the composition is preferably higher than 4.5 but lower than 6.5. Weak acidity in pH is suitable for a living cell so that the active component of the composition can function most effectively. The composition obtained by extracting each raw material for extraction with hot water generally has pH higher than 4.5 but lower than 6.5 as the stock solution.

The composition of the present invention does not exclude a form containing other active components. Other active components can be contained as long as a synergistic effect of two kinds of the extract composition in the composition of the present invention is not hindered. These active components can be an artificially prepared product based on the extraction component or its composition . Disclosure of the present invention particularly intends the active component extracted from a natural product, but naturally intends a chemically synthesized active component to express the effect described herein.

The composition of the present inventionhas an antitumor activity, particularly against leukemia, cervical cancer, lung cancer, ovarian cancer, breast cancer (including metastatic cancer) and skin cancer (including metastatic cancer). The leukemia includes Erythroblastic leukosis. The composition of the present invention can be used as the antitumor drug not only for humans but also for a wide range of mammals such as cows, horses, dogs and cats. The antitumor activity of the extract component from Basidiomycetes has been heretofore known and the antitumor activity of the extract component from the root of a plant belonging to Araliaceae has been confirmed. However, it has already been confirmed that the composition of the present invention shows high antitumor activity beyond expectation as compared with the antitumor activity by that of each single component. The composition of the present invention also has a hypoglycemic effect. This hypoglycemic drug expresses an effect on both insulin-dependent and non-insulin-dependent diabetes mellitus . Furthermore, the composition of the present invention has a hypotensive action against humans and can be used to treat hypertension and serving as its prophylactic. The composition of the present invention also has a total cholesterol-lowering action in blood and neutral fat-lowering action in the blood of humans, etc., and therefore may be used to treat hyperlipidemia and serving as its prophylactic.

In addition, the composition of the present invention has no adverse effect but properties to lower or abolish the side effects of another drug. In particular, when used as the antitumor drug, in addition to healing or degeneration of the tumor, various effects such as pain reduction, appetite improvement and good sleep can be achieved. When used as the hypoglycemic drug, in addition to lowering of the blood glucose level, effects such as palliation of body pain, in particular, remarkable improvement in headaches and numbness of the extremities, appetite enhancement, vision recovery, lowering of stress and comfortable sleep can be achieved.

Extract components from the Basidiomycete fungi and an Araliaceae plant, which are the active components in the present invention are mixed with a pharmaceutically acceptable carrier or additive to be used as the composition suitable for administration. A form of the composition is not particularly limited to, but can be a liquid drug, a syrup, a suspending agent, a powder drug, a granule, a tablet, a pill, a capsule, an emulsion, a troche, a chewable formulation, a suppository, an eye drop, an injectable solution, an aerosol and an elixir. A solid (powder), which can be converted to a liquid formed by adding water when used is also preferable.

The composition of the present invention can also be administered orally or parenterally. Preferably it is orally administered. A typical dose in oral administration is described below. The dose is properly determined according to the symptom and physical strength of an individual. That is, as a typical dose (usual dose) of, for example, an extract component from 200 mg to 2 g of the Basidiomycete fungus and 100 mg to 1 g of a root of an Araliaceae plant per kg body weight per day is administered dividing into one to three times per day. In particular, when used as the antitumor drug, a dose of the extract component from 0.25 g to 0.35 g of both the Basidiomycete fungus and a root of an Araliaceae plant/kg body weight/day is preferable. When used as the hypoglycemic drug, a dose of the extract component from 0.12 to 0.18 g of the Basidiomycete fungus and 0.12 g of the root of Araliaceae plant/kg body weight/day is preferable. When used as the hypoglycemic drug, administration of water and/or an alcohol extract from the root of a plant belonging to Araliaceae is preferably involved. This alcohol extract can be obtained by crushing the root of an Araliaceae plant, preferably a ginseng, more preferably a Japanese ginseng into small pieces or powder (preferably small pieces of a roughly 5 mm of a cubed side) and adding, for example, 300 to 600 parts by weight of a 40% alcohol solution to 10 to 20 parts by weight of the crushed pieces to heat at 80 to 100°C for extraction until the alcohol component is substantially evaporated.

The composition of the present invention is extremely low in toxicity and does not express any serious side effects so that a large dose according to the symptom can be safely administered. A preferred form of the drug for oral administration is a liquid drug or syrup. Water is preferable as a medium.

### Example

The present invention is described below with illustrative examples, but these Examples are not construed to limit the scope of the present invention.

### [Measurement of Oxidation-Reduction Potential of the Composition]

A carpophore of Ganoderma Lucidum, a carpophore of Coriolous Versicolor, a root of Japanese ginseng (P. japonicus C. A. Meyer), a carpophore of the Phellinus linteus and a carpophore of Agaricus blazei Murill (Japanese name, Kawariharatake) were used as a rawmaterial. Every carpophore used was dried.

The raw material was prepared using the composition in Table 1 below and each of them was chopped into a cube shorter than 5 mm on the side or smaller, to which 1000 ml of ion-exchanged water (purity, less than 1 µS/cm) was added and refluxed for two hours after attaching a reflux condenser for extraction. After extraction, the mixture was filtered to yield various formulated solutions. The oxidation-reduction potential and pH of the obtained various formulated solutions were measured at the time when the formulation was prepared as well as the times after the formulation was stored in an incubator at 25°C and relative humidity of 14% for 1, 2. 3 and 4 days. The oxidation-reduction potential and pH were measured with an HM-14P pH meter from TOA Radio Wave Industry Co., Inc. The reported oxidation-reduction potential is based on the value indicated by the measurement instrument to which the value indicated by the reference electrode is added. The results are shown in Tables 2 and 3.

**Table 1**

| Component | Formulated Liquid 1 | Formulated Liquid 2 | Formulated Liquid 3 | Formulated liquid 4 | Formulated Liquid 5 | Formulated Liquid 6 | Formulated Liquid 7 |
|---|---|---|---|---|---|---|---|
| Reishi | 6 | 6 | 6 | 6 | 6 | | |
| Coriolous Versicolor | 6 | 6 | | | 6 | | |
| P.japonicus C.A. Meyer | 6 | 6 | 6 | 6 | 6 | | |
| Phellinus Linteus | 6 | | 6 | | | 6 | |
| Agariousblazei Murill | | 6 | | 6 | | | 6 |

| | | | | | | | |
|---|---|---|---|---|---|---|---|
| Unit: g | | | | | | | |

**Table 2**

| Oxidation-reduction Potential mV | At Time of Preparation | After 1 day | After 2 day | After 3 day | After 4 day |
|---|---|---|---|---|---|
| Formulated Liquid 1 | 297 | 348 | 351 | 332 | 301 |
| Formulated Liquid 2 | 283 | 361 | 288 | 225 | - |
| Formulated Liquid 3 | 257 | 320 | 299 | 228 | 283 |
| Formulated Liquid 4 | 187 | 280 | -301 | - | - |
| Formulated Liquid 5 | 248 | 322 | 398 | 388 | 48 |
| Formulated Liquid 6 | 304 | 362 | 274 | 295 | 330 |
| Formulated Liquid 7 | 219 | 272 | 250 | 92.5 | -321 |

**Table 3**

| pH | At Time of Preparation | After 1 day | After 2 day | After 3 day | After 4 day |
|---|---|---|---|---|---|
| Formulated Liquid 1 | 4.67 | 4.64 | 4.57 | 4.53 | 4.57 |
| Formulated Liquid 2 | 5.52 | 5.47 | 5.61 | 5.75 | - |
| Formulated Liquid 3 | 5.19 | 5.14 | 5.12 | 4.84 | 4.75 |
| Formulated Liquid 4 | 5.91 | 6.36 | 5.71 | - | - |
| Formulated Liquid 5 | 5.05 | 5.07 | 5.1 | 5.07 | 5.19 |
| Formulated Liquid 6 | 4.43 | 4.4 | 4.34 | 4.37 | 4.42 |
| Formulated Liquid 7 | 6.2 | 6.18 | 6.18 | 5.91 | 5.6 |

As shown in Table 2, each of the formulated liquid 1 (RKGME), formulated liquid 2 (RKGA), formulated liquid 3 (RMEG) and formulated liquid 4 (RAG) has a characteristic value in the oxidation-reduction potential. The oxidation-reduction potential of both formulated liquids 1 and 3 including an extract component from the Phellinus linteus shows a virtually constant value regardless of the passage of time. On the other hand, the oxidation-reduction potential of the formulated liquids 2 and 4 including the extract component from Agarics remarkably decreases with time. The oxidation-reduction potential of the formulated liquid 5 (RKG) decreases with time, but stays in the middle between that of the extract component from Phellinus linteus and that of the extract component from Agarics. On the other hand, the oxidation-reduction potential of the formulated liquid 6 (extracted liquid from Phellinus linteus alone) stays constant with time, while that of the formulated liquid 7 (extracted liquid from Agarics alone) remarkably decreases with time. It can be concluded from the results that a change in the oxidation-reduction potential over time in the formulated liquids 1 and 3 containing Phellinus linteus and the formulated liquids 2 and 4 containing Agarics corresponds to a change in the oxidation-reduction potential over time in the formulated liquid 6 (extracted liquid from Phellinus linteus alone) and the formulated liquid 7 (extracted liquid from Agarics alone), respectively.

On the other hand, as shown in Table 3, pH is nearly stable over time in all formulated liquids- pH of the formulated liquids 1 to 4 at the time for preparing is weakly acidic within a range higher than 4.5 but lower than 6.0, the value being maintained during the shelf period. The present inventor confirmed the formulated liquid 5 (RGK) has the antitumor effect, hypoglycemic effect and cholesterol-lowering effect as well as revealed these physiological activities can be associated with the value of oxidation-reduction potential lower than 330 mV (U.S. Patent No. 6.746,675). A full text of the description in U.S. Patent No. 6,746,675 is a part of the present description by quotation.

A fact that the formulated liquids 1 to 4 can maintain their oxidation-reduction potentials or a reduced oxidation-reduction potential without significant increase at least against the passage of time (that is, against oxidation) possibly indicates these formulated liquids have a reducing power.

It is proved from the results that the formulated liquids 1 to 4 are weakly acidic, do not impair a living cell and possess the oxidation-reduction potential lower than 330 mV at a time when the solution is prepared, so that the physiological activity such as the antitumor effect and hypoglycemic effect is recognized.

### [Further Typical Method for Preparing the Composition of the Present Invention]

In the Example, the hot water extract from the Basidiomycete fungus and that from the root of a plant belonging to Araliaceae are used, but other compositions can be intended. For example, a component can be extracted from a natural product with other extraction methods . As an example, a starting raw material is subjected to a certain extraction process to filter the extract component, which is evaporated under reduced pressure. The product is sterilized, dried, and then sieved to yield a powder.

The dried extract composition is prepared to a powder, a granule, a capsule and a tablet, etc. For example, the extraction component is mixed with a binder to granulate and dry to yield a granule. The granule may be used as it is, converted to a tablet, or filled into a capsule. An additional binder can be added either before or after the granulation process of the extract component after drying. The extract component can be dried to fill a capsule for encapsulation. Other pharmaceutically acceptable additives can also be added. The additive is not limited to, but includes one or more than two kinds of preservatives to extend a half life of the component.

Furthermore, an effective component can be dissolved in a pharmaceutically acceptable solvent and filtered to be filled in an ampoule. This ampoule can be sterilized. Furthermore, other pharmaceutically acceptable additives can be added to the solution. The additive is not limited to, but includes one or more than two kinds of preservatives to extend a half life of the component.

After separation of an effective component, the isolated effective component can be singly used as a therapeutic agent. The effective component can be identified and prepared in a chemically synthetic manner. In this case, the chemically synthesized effective component can be used in a form of a powder, a granule, a capsule, a tablet or an ampoule, etc., and provide other mediums to administer the composition to a patient. Other pharmaceutically acceptable effective additives can be added if needed.

## Claims

1. A physiologically active composition containing:
an extract component from a carpophore of the following Basidiomycete fungi: Ganoderma lucidum, Coriolus Versicolor and Phellinus linteus; and
an extract component from a root of P. japonicus C.A. Meyer belonging to Araliaceae, wherein the ratio of Ganoderma Lucidum:Coriolus Versicolor:Phellinus Linteus:P. japonicus C.A. Meyer in the raw material for extraction is 1:1:1:1 by weight, wherein the extract composition from a carpophore of the Basidiomycete fungi and the extract component from a root of P. japonicusC.A. Meyerare respectively extract components with hot water, and wherein the oxidation-reduction potential of an aqueous solution of the composition is not more than 330 mV.

2. The composition according to Claim 1, wherein pH of an aqueous solution of the composition is not less than 4.5 and not more than 6.5.

3. A drug formulation which is mixed when used and is provided with a first drug comprising an extract component from a carpophore of Ganoderma lucidum, Coriolus Versicolor, and Phellinus Linteus, and a second drug comprising an extract component from a root of P. japonicus C.A. Meyer, wherein the ratio of Ganoderma Lucidum:Coriolus Versicolor:Phellinus Linteus:P. japonicus C.A. Meyer in the raw material for extraction is 1:1:1:1 by weight, wherein the extract composition from a carpophore of the Basidiomycete fungi and the extract component from a root of P. japonicus C.A. Meyer are respectively extract components with hot water, and wherein the oxidation-reduction potential of an aqueous solution of the formulation is not more than 330 mV.

4. A preparing method of a physiologically active composition according to any one of claims 1 to 3, said method comprising the steps of;
extracting with hot water a carpophore of Ganoderma lucidum and Coriolus Versicolor;
extracting with hot water a carpophore of Phellinus linteus; extracting a root of P. japonicus C.A. Meyer with hot water;
wherein the ratio of Ganoderma Lucidum: Coriolus Versicolor:Phellinus Linteus:P. japonicus C.A. Meyer in the raw material for extraction is 1:1:1:1 by weight and
mixing the extract components with the hot water obtained in the extracting steps.

5. A preparing method of a physiologically active composition according to any one of claims 1 to 3, said method comprising the steps of:
obtaining a hot water extract from a carpophore of Ganoderma lucidum and Coriolus Versicolor, a hot water extract from a carpophore of Phellinus linteus, and a hot water extract from a root of P. japonicus C.A. Meyer separately or from the same extraction system with respect to at least more than two kinds of the hot water extracts, so as to prepare a composition comprising the hot water extract component from a carpophore of a fungus belonging to Ganoderma lucidum and Coriolus Versicolor, the hot water extract component from a carpophore of Phellinus linteus and the hot water extract component from a root of P. japonicus C.A. Meyer, wherein the ratio of Ganoderma Lucidum: Coriolus Versicolor:Phellinus Linteus:P. japonicus C.A. Meyer in the raw material for extraction is 1:1:1:1 by weight.

## Patentansprüche

1. Physiologisch aktive Zusammensetzung enthaltend:
einen Extraktbestandteil eines Karpophors der folgenden Basidiomyceten-Pilze: Ganoderma lucidum, Coriolus versicolor und Phellinus linteus; und
einen Extraktbestandteil einer Wurzel von P. japonicus C. A. Meyer, zugehörig zu den Araliaceae, wobei das Gewichtsverhältnis von Ganoderma lucidum : Coriolus versicolor : Phellinus linteus : P. japonicus C. A. Meyer in dem Rohmaterial für die Extraktion 1 : 1 : 1 : 1 beträgt, wobei die Extraktzusammensetzung eines Karpophors der Basidiomyceten-Pilze und der Extraktbestandteil einer Wurzel von P. japonicus C. A. Meyer jeweils Extraktbestandteile mit heißem Wasser sind, und wobei das Oxidations-Reduktionspotential einer wässrigen Lösung der Zusammensetzung nicht mehr als 330 mV beträgt.

2. Zusammensetzung gemäß Anspruch 1, wobei der pH einer wässrigen Lösung der Zusammensetzung nicht weniger als 4,5 und nicht mehr als 6,5 beträgt.

3. Arzneiformulierung, welche, wenn sie verwendet wird, gemischt ist und mit einer ersten Arznei, umfassend einen Extraktbestandteil eines Karpophors von Ganoderma lucidum, Coriolus versicolor und Phellinus linteus, und einer zweiten Arznei, umfassend einen Extraktbestandteil einer Wurzel von P. japonicus C. A. Meyer, zur Verfügung gestellt wird, wobei das Gewichtsverhältnis von Ganoderma lucidum : Coriolus versicolor : Phellinus linteus : P. japonicus C. A. Meyer in dem Rohmaterial für Extraktion 1 : 1 : 1 : 1 beträgt, wobei die Extraktzusammensetzung eines Karpophors der Basidiomyceten-Pilze und der Extraktbestandteil einer Wurzel von P. japonicus C. A. Meyer jeweils Extraktbestandteile mit heißem Wasser sind, und wobei das Oxidations-Reduktionspotential von einer wässrigen Lösung der Formulierung nicht mehr als 330 mV beträgt.

4. Herstellungsverfahren einer physiologisch aktiven Zusammensetzung gemäß einem der Ansprüche 1 bis 3, wobei dieses Verfahren die Schritte umfasst:
Extrahieren eines Karpophors von Ganoderma lucidum und Coriolus versicolor mit heißem Wasser;
Extrahieren eines Karpophors von Phellinus linteus mit heißem Wasser;
Extrahieren einer Wurzel von P. japonicus C. A. Meyer mit heißem Wasser;
wobei das Gewichtsverhältnis von Ganoderma lucidum :
Coriolus versicolor : Phellinus linteus : P. japonicus C. A. Meyer in dem Rohmaterial für die Extraktion 1 : 1 : 1 : 1 beträgt, und
Mischen der Extraktbestandteile mit dem in den Extraktionsschritten erhaltenen heißen Wasser.

5. Herstellungsverfahren einer physiologisch aktiven Zusammensetzung gemäß einem der Ansprüche 1 bis 3, wobei dieses Verfahren die Schritte umfasst:
Erhalten eines Heißwasserextrakts eines Karpophors von Ganoderma lucidum und Coriolus versicolor, eines Heißwasserextrakts eines Karpophors von Phellinus linteus und eines Heißwasserextrakts einer Wurzel von P. japonicus C. A. Meyer, getrennt oder aus dem gleichen Extraktionssystem in Bezug auf mindestens mehr als zwei Arten der Heißwasserextrakte, um so eine Zusammensetzung herzustellen, die den Heißwassersextraktbestandteil eines Karpophors von einem Pilz, zugehörig zu Ganoderma lucidum und Coriolus versicolor, den Heißwasserextraktbestandteil eines Karpophors von Phellinus linteus, und den Heißwasserextraktbestandteil einer Wurzel von P. japonicus C. A. Meyer umfasst, wobei das Gewichtsverhältnis von Ganoderma lucidum : Coriolus versicolor : Phellinus linteus : P. japonicus C. A. Meyer in dem Rohmaterial für die Extraktion 1 : 1 : 1 : 1 beträgt.

## Revendications

1. Composition physiologiquement active contenant :
un composant extrait à partir d'un sporophore des champignons Basidiomycètes suivants : Ganoderma lucidum, Coriolus Versicolor et Phellinus linteus; et
un composant extrait à partir d'une racine de P. japonicus C.A. Meyer appartenant à la famille des Araliacées, où le rapport de Ganoderma Lucidum :
Coriolus Versicolor : Phellinus Linteus : P. japonicus C.A. Meyer dans le matériau brut pour l'extraction est de 1 : 1 : 1 : 1 en poids, où la composition extraite à partir d'un sporophore des champignons Basidiomycètes et le composant extrait à partir d'une racine de P. japonicus C.A. Meyer sont respectivement des composant extraits avec de l'eau chaude, et où le potentiel d'oxydo-réduction d'une solution aqueuse de la composition n'est pas supérieur à 330 mV.

2. Composition selon la revendication 1, où le pH d'une solution aqueuse de la composition n'est pas inférieur à 4,5 et n'est pas supérieur à 6,5.

3. Formulation de médicament qui est mélangée quand elle est utilisée et qui est munie d'un premier médicament comprenant un composant extrait à partir d'un sporophore de Ganoderma lucidum, Coriolus Versicolor, et Phellinus Linteus, et un deuxième médicament comprenant un composant extrait à partir d'une racine de P. japonicus C.A. Meyer, où le rapport de Ganoderma Lucidum : Coriolus Versicolor : Phellinus Linteus : P. japonicus C.A. Meyer dans le matériau brut pour l'extraction est de 1 : 1 :
1 : 1 en poids, où la composition extraite à partir d'un sporophore des champignons Basidiomycètes et le composant extrait à partir d'une racine de P. japonicus C.A. Meyer sont respectivement des composants extraits avec de l'eau chaude, et où le potentiel d'oxydo-réduction d'une solution aqueuse de la formulation n'est pas supérieur à 330 mV.

4. Procédé de préparation d'une composition physiologiquement active selon l'une quelconque des revendications 1 à 3, ledit procédé comprenant les étapes consistant à :
extraire avec de l'eau chaude un sporophore de Ganoderma lucidum et Coriolus Versicolor;
extraire avec de l'eau chaude un sporophore de Phellinus linteus;
extraire une racine de P. japonicus C.A. Meyer avec de l'eau chaude ;
où le rapport de Ganoderma Lucidum : Coriolus Versicolor : Phellinus Linteus : P. japonicus C.A. Meyer dans le matériau brut pour l'extraction est de 1 : 1 : 1 : 1 en poids et
mélanger les composants extraits avec de l'eau chaude obtenue dans les étapes d'extraction.

5. Procédé de préparation d'une composition physiologiquement active selon l'une quelconque des revendications 1 à 3, ledit procédé comprenant les étapes consistant à :
obtenir un extrait à l'eau chaude à partir d'un sporophore de Ganoderma lucidum et de Coriolus Versicolor, un extrait à l'eau chaude à partir d'un sporophore de Phellinus linteus, et un extrait à l'eau chaude à partir d'une racine de P. japonicus C.A.
Meyer séparément ou à partir du même système d'extraction par rapport à au moins plus de deux types d'extraits à l'eau chaude, de manière à préparer une composition comprenant le composant extrait à l'eau chaude à partir d'un sporophore d'un champignon appartenant aux espèces Ganoderma lucidum et Coriolus Versicolor, le composant extrait à l'eau chaude à partir d'un sporophore de Phellinus linteus et le composant extrait à l'eau chaude à partir d'une racine de P. japonicus C. A. Meyer, où le rapport de Ganoderma Lucidum : Coriolus Versicolor : Phellinus Linteus : P. japonicus C.A. Meyer dans le matériau brut pour l'extraction est de 1 : 1 : 1 : 1 en poids.
